# EUROPÄISCHE PATENTSCHRIFT

(11) **EP 3 673 075 B1**
(45) Veröffentlichungstag und Bekanntmachung des Hinweises auf die Patenterteilung: **27.10.2021**
(21) Anmeldenummer: 18752516.7
(22) Anmeldetag: 16.08.2018
(51) Int. Cl.: C12Q 1/04, C12Q 1/6818, C12Q 1/6841

(54) **VERFAHREN ZUM SPEZIFISCHEN NACHWEIS VON MIKROORGANISMEN**
METHOD FOR SPECIFIC DETECTION OF MICROORGANISMS
PROCÉDÉ DE DÉTECTION SPÉCIFIQUE DE MICRO-ORGANISMES

(30) Priorität: 22.08.2017 EP 17187339
(43) Veröffentlichungstag der Anmeldung: 01.07.2020
(73) Patentinhaber: Vermicon AG, 80992 München (DE)
(72) Erfinder: SNAIDR, Jiri, 80992 München (DE); BEIMFOHR, Claudia, 80992 München (DE); MÜHLHAHN, Peter, 80992 München (DE)
(74) Vertreter: Henkel & Partner mbB
(86) Internationale Anmeldenummer: PCT/EP2018/072240
(87) Internationale Veröffentlichungsnummer: WO 2019/038181

(56) Entgegenhaltungen:
- WO-A1-03/083131
- WO-A1-2016/178953
- DE-A1-102010 012 421
- DOUGLAS P. SHEPHERD ET AL: "Counting Small RNA in Pathogenic Bacteria", ANALYTICAL CHEMISTRY, Bd. 85, Nr. 10, 25. April 2013 (2013-04-25), Seiten 4938-4943, XP055436589, US ISSN: 0003-2700, DOI: 10.1021/ac303792p
- MORRISON L E ET AL: "Solution-phase detection of polynucleotides using interacting fluorescent labels and competitive hybridization", ANALYTICAL BIOCHEMISTRY, ELSEVIER, AMSTERDAM, NL, Bd. 183, Nr. 2, 1. Dezember 1989 (1989-12-01), Seiten 231-244, XP024820477, ISSN: 0003-2697, DOI: 10.1016/0003-2697(89)90473-9 [gefunden am 1989-12-01]
- HYUNGSEOK C MOON ET AL: "Tracking single mRNA molecules in live cells", JOURNAL OF PHYSICS D: APPLIED PHYSICS, INSTITUTE OF PHYSICS PUBLISHING LTD, GB, Bd. 49, Nr. 23, 6. Mai 2016 (2016-05-06), Seite 233001, XP020305336, ISSN: 0022-3727, DOI: 10.1088/0022-3727/49/23/233001 [gefunden am 2016-05-06]

## Beschreibung

Die vorliegende Erfindung betrifft ein Verfahren zum spezifischen Nachweis eines Mikroorganismus oder einer Gruppe von Mikroorganismen durch in-situ-Hybridisierung mittels Durchflusszytometrie, sowie die Verwendung eines Durchflusszytometers in diesem Verfahren.

Die mikrobielle Routineanalytik in der Lebensmittelindustrie ist in vielen Bereichen mit einem hohen Probenaufkommen (> 100 Proben pro Tag) konfrontiert, welches schnell und möglichst parallel abgearbeitet werden muss. Um die Verarbeitung eines derartig großen Probendurchsatzes (Highthroughput) mit einem spezifischen, molekularbiologischen Schnellnachweissystem zu realisieren, bieten sich Systeme mit automatischer Auswertung und objektiver Ergebnisermittlung an.

Als moderne Methode der Bakterienbestimmung, die geeignet ist, diese Anforderungen zu erfüllen, hat sich mittlerweile die sogenannte Fluoreszenz *in situ* Hybridisierung (FISH) etabliert, in deren ursprünglicher Ausführungsform Zellen auf einen Objektträger durch fluoreszenzmarkierte Nukleinsäuresondenmoleküle unter dem Mikroskop sichtbar gemacht werden (Amann et al., Microbial. Rev. 59 (1995), 143-169). Allerdings ist die klassische FISH-Technik insbesondere mit dem Problem behaftet, dass die Detektion mittels Mikroskop gut geschultes Laborpersonal und einen erheblichen Zeitaufwand erfordert, was die Anzahl der pro Tag analysierten Proben naturgemäß stark begrenzt.

Um diesem Problem zu begegnen, wurde in den darauffolgenden Jahren eine auf dem Reaktionsmechanismus der klassischen FISH-Technik basierende Fluoreszenz-in-situ-Hybridisierung in flüssiger Phase vorgeschlagen, in welcher alle Schritte der Hybridisierungsreaktion nahezu unverändert vom Format Objektträger auf das Format Reaktionsgefäß übertragen wurden und eine Detektion mittels Durchflusszytometer erfolgte (WO 03/083131 A1). Im Rahmen dieses Verfahrens wurden die verschiedenen Hybridisierungs- und Waschlösungen via Zentrifugation entfernt.

Insbesondere die bei der Ganzzellhybridisierung erforderlichen Waschschritte zur Erhöhung des Signal-Rausch-Verhältnisses in der Routineanalytik sind indessen sehr arbeitsintensiv und erfordern in der Handhabung ebenfalls gut geschultes Laborpersonal. Weiterhin stellen die bei der Zentrifugation notwendigen Schritte zur Entfernung der wässrigen Überstände einen weiteren Prozessparameter dar, der bei der Würdigung des beobachteten Ergebnisses und der erforderlichen Standardisierung des Verfahrens zu beachten ist.

DE 10 2010 012 421 A1 offenbart ein Verfahren zum spezifischen Nachweis von Mikroorganismen, welches schnell durchgeführt werden kann und ohne die bei der klassischen FISH-Technik erforderlichen Waschschritte auskommt. Im Einzelnen umfasst das Verfahren eine Durchführung der Hybridisierungsreaktion in einer Mikrotiterplatte sowie die anschließende Ergebnisermittlung über einen Mikrotiterplattenleser, wobei das ausgelesene Fluoreszenzsignal der Summe der an den Mikroorganismen spezifisch gebundenen fluoreszenzmarkierten Nukleinsäuresonden entspricht.

Eine maßgebliche Voraussetzung für diese semiquantitative Bakterienbestimmung besteht in der spezifischen Fluoreszenzauslöschung von ungebundenen fluoreszenzmarkierten Nukleinsäuresondenmolekülen innerhalb des Reaktionsraums der Mikrotiterplatte, um eine Diskriminierung von spezifischen zu unspezifischen Signalen zu ermöglichen. Das Verfahren hat indessen den Nachteil, dass eine präzise Quantifizierung der Zellzahl nicht gegeben ist, da die Anzahl an spezifisch gebundenen fluoreszenzmarkierten Nukleinsäuresondenmolekülen pro Zelle (und damit der Beitrag einer Zelle zur gemessenen Fluoreszenzsignalintensität) nicht eindeutig bestimmbar ist. Eine einzelne Zelle kann dabei in der Regel zwischen 5000 bis 15000 fluoreszenzmarkierte Oligonukleotidsonden binden.

Shepherd et al. (Anal. Chem. 85 (2013), 4938-4943) lehrt ein Verfahren zur Quantifizierung von sRNA aus pathogenen Bakterien mittels einer Kombination aus smFISH-Technik und nachfolgender Fluoreszenzauslöschung. Das Verfahren umfasst das Fixieren von Zellen aus Yersinia pestis oder Yersinia tuberculosis mit Formaldehyd, das Permeabilisieren der fixierten Zellen mit 70%-igem Ethanol, das Inkontaktbringen und Inkubieren der permeabilisierten Zellen mit einem Hybridisierungspuffer, welcher eine Vielzahl an Yersiniaspezifischen, fluoreszenzmarkierten Nukleinsäuresonden enthält, das Abfangen von ungebundenen fluoreszenzmarkierten Nukleinsäuresonden durch Inkontaktbringen und Inkubieren des Reaktionsgemischs mit einer Lösung einer Vielzahl von hierzu komplementären, quenchermarkierten Nukleinsäuresonden, und das Detektieren der Fluoreszenz einer auf diese Weise erhaltenen Probe unter Verwendung eines Spektralphotometers.

Der vorliegenden Erfindung lag daher die Aufgabe zugrunde, ein Verfahren zum Nachweis von Mikroorganismen allgemein und eines Mikroorganismus im speziellen bereitzustellen, welches die vorstehenden Nachteile des Standes der Technik überwindet. Insbesondere sollte das Verfahren möglichst einfach (d.h. mit möglichst geringem technischem Aufwand und auch in Abwesenheit von geschultem Laborpersonal) durchgeführt werden können und die Möglichkeit bieten, innerhalb kurzer Zeit eine große Menge an Proben zu analysieren und gleichzeitig eine hohe Spezifität für probenrelevante Mikroorganismen zu gewährleisten. Dabei sollte die Ergebnisermittlung und Beurteilung objektiv und standardisiert erfolgen. Die Erfindung ist durch die Ansprüche definiert.

In einem ersten Aspekt zur Lösung der Aufgabe betrifft die Erfindung somit ein Verfahren, wie es in Anspruch 1 definiert ist. Das erfindungsgemäße Verfahren erlaubt den schnellen und spezifischen Nachweis von Mikroorganismen, ohne dass es dabei eines Waschschrittes wie bei der klassischen FISH-Technik bedarf. Daneben wird mit dem erfindungsgemäßen Verfahren die bei der FISH-Technik, und insbesondere der klassischen FISH-Technik auftretende und bei der Auswertung nicht unproblematische unspezifische Autofluoreszenz unterdrückt.

Weiterhin ermöglicht das erfindungsgemäße Verfahren im Vergleich zu dem in DE 10 2010 012 421 A1 beschriebenen Mikrotiterplattenverfahren eine stark vereinfachte Durchführung, indem die herkömmlichen Verfahrensschritte auf die Zugabe von 2 Reaktionslösungen zum Probenansatz verkürzt werden und die Reaktion in einem einzigen Reaktionsgefäß erfolgen kann. Zudem wird durch die Verwendung eines Durchflusszytometers im Rahmen der Detektion eine direkte Quantifizierung der detektierten Mikroorganismen möglich.

Schließlich kann das vorliegende Verfahren zumindest und auch hinsichtlich der Durchführung und Auswertung automatisiert werden. Das erfindungsgemäße Verfahren ist dadurch auch besonders für eine Analytik mit hohem Probendurchsatz (Highthroughput) geeignet.

Die Durchführung des erfindungsgemäßen Verfahrens zum spezifischen Nachweis eines Mikroorganismus oder mehrerer Mikroorganismen in einer Probe umfasst die folgenden Schritte:
(a) Bereitstellen der Probe;
(b) Fixieren der in der Probe enthaltenen Zellen mit einem Fixiermittel und Abtrennen der hierbei erhaltenen fixierten Zellen aus der Probe, um fixierte Zellen bereitzustellen;
(c) Inkontaktbringen der fixierten Zellen mit einem chemischen Homogenisierungsmittel und Trocknen der hierbei erhaltenen homogenisierten Zellen, um getrocknete Zellen bereitzustellen, wobei das chemische Homogenisierungsmittel (i) ein Monosaccharid oder Disaccharid, (ii) ein Polyol, und (iii) Wasser umfasst;
(d) Inkontaktbringen der getrockneten Zellen mit einer Lösung einer für den nachzuweisenden Mikroorganismus spezifischen, fluoreszenzmarkierten Nukleinsäuresonde, um ein erstes Reaktionsgemisch bereitzustellen;
(e) Inkubieren des ersten Reaktionsgemischs, um eine Bindung der fluoreszenzmarkierten Nukleinsäuresonde an die korrespondierende Zielnukleinsäuresequenz in den Zellen des nachzuweisenden Mikroorganismus zu bewirken;
(f) Inkontaktbringen des ersten Reaktionsgemischs im Anschluss an Schritt (e) mit einer Lösung einer quenchermarkierten Nukleinsäuresonde, um ein zweites Reaktionsgemisch bereitzustellen, wobei die quenchermarkierte Nukleinsäuresonde einen die Fluoreszenz der fluoreszenzmarkierten Nukleinsäuresonde zumindest teilweise löschenden Quencher umfasst und eine Nukleinsäuresequenz aufweist, welche im Wesentlichen komplementär zu der Nukleinsäuresequenz der fluoreszenzmarkierten Nukleinsäuresonde ist;
(g) Inkubieren des zweiten Reaktionsgemischs, um eine Bindung der nicht an die Zielnukleinsäuresequenz in den Zellen des nachzuweisenden Mikroorganismus gebundenen Moleküle der fluoreszenzmarkierten Nukleinsäuresonde an die quenchermarkierte Nukleinsäuresonde zu bewirken; und
(h) Einbringen des zweiten Reaktionsgemischs im Anschluss an Schritt (g) in ein Durchflusszytometer, und Detektieren der Fluoreszenz, welche von den die fluoreszenzmarkierte Nukleinsäuresonde beinhaltenden Zellen des nachzuweisenden Mikroorganismus emittiert wird.

Die vorliegende Erfindung betrifft den spezifischen Nachweis eines einzelnen Mikroorganismus oder mehrerer (d.h. mindestens zweier unterschiedlicher) Mikroorganismen in einer natürlich vorkommenden oder künstlich zusammengestellten Probe. Es versteht sich, dass beim Nachweis eines derartigen Mikroorganismus im Rahmen des erfindungsgemäßen Verfahrens mehr als nur eine einzelne Zelle nachgewiesen wird. In der Regel erfolgt der Nachweis einer oder mehrerer Zellen, wobei der Nachweis auf dem Nachweis der von einer Einzelzelle ausgehenden Fluoreszenz beruht.

Der Begriff "Mikroorganismus", wie er im Rahmen der vorliegenden Anmeldung verwendet wird, umfasst sowohl natürlich vorkommende als auch künstlich erzeugte Mikroorganismen, welche pathogener oder apathogener Natur sein können und u.a. Bakterien, Pilze, Mikroalgen und Protozoen beinhalten. Vorzugsweise handelt es sich bei dem mindestens einen nachzuweisenden Mikroorganismus um ein Bakterium, einen Pilz oder einen einzelligen höheren Organismus (Protozoon), wobei das Bakterium, der Pilz oder/und der einzellige höhere Organismus einer beliebigen taxonomischen Einheit entstammen kann und der Begriff "taxonomische Einheit" u.a. Domänen/Königreiche, Divisionen/Phyla, Klassen, Subklassen, Ordnungen, Subordnungen, Familien, Subfamilien, Gattungen, Untergattungen, Arten, Unterarten, Stämme und Unterstämme umfasst.

Konkrete Beispiele für Mikroorganismen, welche mittels des erfindungsgemäßen Verfahrens nachgewiesen werden können, umfassen insbesondere Bakterien, Pilze (umfassend Hefen und Schimmelpilze) und Protozoen, welche bekanntermaßen die Qualität von Wasser (einschließlich Abwasser), Getränken (z.B. Wasser, Bier, Fruchtsäften und nichtalkoholischen Erfrischungsgetränken) und Lebensmitteln (z.B. Milchprodukten wie Käse und Joghurt, sowie Fleischprodukten wie Wurstwaren) schädigen und u.a. in DE 101 29 410 A1, DE 101 60 666 A1 und WO 2005/031004 A2 genannt sind. Der Nachweis von Bakterien, Hefen und Schimmelpilzen ist in diesem Zusammenhang besonders bevorzugt.

Als probenrelevante Bakterien gelten hierbei insbesondere Bakterien der Gattungen Acetobacter, Achromobacter, Acinetobacter, Aerococcus, Aeromonas, Agrobacterium, Alcaligenes, Alicyclobacillus, Aneurinibacillus, Anoxybacillus, Aquabacterium, Arcobacter, Arthrobacter, Arthrobacter, Bacillus, Brevibacillus, Brevibacterium, Brocardia, Brochothrix, Burkholderia, Caldanaerobius, Campylobacter, Carnobacterium, Cellulomonas, Chloroflexi, Chryseobacterium, Chryseobacterium, Citrobacter, Cloacibacterium, Clostridium, Colwellia, Corynebacterium, Cronobacter, Delftia, Desulfotomaculum, Dickeya, Enterobacter, Enterobacteriaceae, Enterococcus, Erwinia, Escherichia, Facklamia, Flavobacteriaceae, Flavobacterium, Fructobacillus, Geobacillus, Gluconacetobacter, Gluconobacter, Janthinobacterium, Jeotgalibacillus, Kocuria, Komagtaeibacter, Kuenenia, Kurthia, Lactobacillus, Lactococcus, Legionella, Lentibacillus, Leuconostoc, Listeria, Lysinibacillus, Macrococcus, Marinilactibacillus, Megasphaera, Microbacterium, Micrococcus, Microthrix, Milchsäurebakterien, Moorella, Moraxella, Nitrobacter, Nitrosococcus, Nitrosomonas, Nitrospira, Nitrotoga, Nocardia, Nostocoida, Obesumbacterium, Oceanobacillus, Oenococcus, Paenibacillus, Pantoea, Pectinatus, Pectobacterium, Pediococcus, Pedobacter, Photobacterium, Prevotella, Propionibacterium, Pseudoalteromonas, Pseudomonas, Psychrobacillus, Psychrobacter, Psychroflexus, Rhizobium, Salmonella, Scalindua, Serratia, Shewanella, Shigella, Solibacillus, Sphingobacterium, Sphingomonas, Sporolactobacillus, Sporosarcina, Staphylococcus, Stenotrophomonas, Streptococcus, Streptomyces, Tepidomonas, Thermoanaerobacterium, Thermophile Bakterien, Thiothrix, Trichococcus, Ureibacillus, Vagococcus, Vibrio, Virgibacillus, Viridibacillus, Weissella, Xanthomonas, Yersinia und Zymomonas.

Probenrelevante Pilze, welche mittels des Verfahrens der vorliegenden Erfindung nachgewiesen werden können, umfassen insbesondere Schimmelpilze und Hefen der Gattungen Aspergillus, Candida, Debaromyces, Dekkera, Geotrichum, Hanseniaspora, Hyphopichia, Kazachstania, Kloeckera, Kluyveromyces, Lodderomyces, Penicillium, Pichia, Rhodotorula, Saccharomyces, Saccharomycopsis, Schizosaccharomyces, Torulaspora, Wickerhamomyces, Yarrowia und Zygosaccharomyces.

Probenrelevante einzellige höhere Organismen (Protozoen), welche mittels des Verfahrens der vorliegenden Erfindung nachgewiesen werden können, umfassen insbesondere Giardia, Cryptosporidium, Amöba, Trichomonas, Toxoplasma, Balantidium und Blastocystis.

Was den Nachweis von Mikroorganismen in Wasser, Getränken und Lebensmitteln betrifft, so sind Bakterien der Gattungen Acinetobacter, Alicyclobacillus, Aquabacterium, Arcobacter, Bacillus, Campylobacter, Enterobacteriaceae, Escherichia, Lactobacillus, Lactococcus, Legionella, Listeria, Microthrix, Nitrobacter, Nitrosococcus, Nitrosomonas, Nitrospira, Nitrotoga, Propionibacterium, Salmonella, Shigella und Streptococcus, sowie Pilze der Gattungen Aspergillus, Candida, Debaromyces, Dekkera, Penicillium, Pichia und Saccharomyces von besonderer Relevanz, weshalb deren Nachweis als besonders bevorzugt gilt.

Die Probe, wie sie im Rahmen des erfindungsgemäßen Verfahrens verwendet wird, ist vorzugsweise eine flüssige Probe. Dabei ist es ausreichend, wenn zumindest ein Teil der einzelnen Zellen des nachzuweisenden Mikroorganismus in der flüssigen Phase vorliegt. Insoweit kann erfindungsgemäß auch eine nur teilweise flüssige Probe oder eine Suspension oder eine Dispersion verwendet werden, wobei eine Lösung als bevorzugt gilt. Besonders bevorzugt wird als flüssige Probe eine wässrige Probe eingesetzt, wie beispielsweise eine Wasserprobe oder eine Getränkeprobe, welche als solche (d.h. ohne Zugabe weiterer Flüssigkeit) einer Analyse zugeführt wird.

Die im Rahmen der Durchführung des erfindungsgemäßen Verfahrens zu analysierende Probe kann dabei jegliche Primärprobe sein, von der eine Sekundärprobe erzeugt wird, die dann als flüssige Probe in dem erfindungsgemäßen Verfahren verwendet wird. Eine Primärprobe kann eine feste, pastöse, flüssige oder gasförmige Probe sein. Typischerweise wird von der Primärprobe eine repräsentative Mischung von Mikroorganismen erhalten, die dann in die flüssige Probe, wie sie im Rahmen des erfindungsgemäßen Verfahrens verwendet wird, überführt wird.

Nachdem eine zu analysierende Probe bereitgestellt worden ist, werden die Zellen des in der Probe enthaltenen Mikroorganismus oder der in der Probe enthaltenen Mikroorganismen im Rahmen des erfindungsgemäßen Verfahrens unter Einsatz eines Fixiermittels fixiert. Der Begriff "Fixierung", wie er im Rahmen der vorliegenden Anmeldung verwendet wird, ist im Bereich der Zellbiologie wohlbekannt und bezeichnet allgemein eine Konservierung biologischer Proben für nachfolgende Untersuchungen. Ein Verfahren zur Fixierung von Zellen, welches im Rahmen von FISH-Techniken zur Anwendung gelangen kann, ist beispielsweise in Amann et al. (Nature Reviews Microbiology 6 (2008), 339-350) beschrieben.

Infolge der Diversität der Zellhüllsysteme und insbesondere auch der Zellwände der verschiedenen nachzuweisenden Mikroorganismen werden die Reaktionsbedingungen für die Fixierung vorzugsweise an den jeweils nachzuweisenden Mikroorganismus adaptiert. Als Fixiermittel wird im Rahmen der vorliegenden Erfindung vorzugsweise ein Alkohol, insbesondere ein kurzkettiger Alkohol wie Methanol, Ethanol oder Isopropanol, oder ein Aldehyd, insbesondere ein kurzkettiger Aldehyd wie Formaldehyd oder para-Formaldehyd, verwendet. Die genauen Reaktionsbedingungen eines Fixierverfahrens unter Einsatz derartiger Fixiermittel können von einem Fachmann auf dem Gebiet der mikrobiellen Diagnostik durch einfache Standardversuche und Routineexperimente bestimmt werden.

Erfindungsgemäß erfolgt die Fixierung der in der Probe enthaltenen Zellen vor dem Inkontaktbringen der Zellen mit dem für den jeweiligen Mikroorganismus spezifischen Nachweisreagenz. Dabei gilt es zum einen, die Integrität und, in einem gewissen Umfang, auch die Form der nachzuweisenden Zellen beizubehalten sowie einen Verlust an Zellen des nachzuweisenden Mikroorganismus insbesondere durch Lyse zu vermeiden. Andererseits gilt es, so viele Zellen wie möglich des nachzuweisenden Mikroorganismus durch das Fixieren zu permeabilisieren, so dass die in dem Nachweisreagenz enthaltenen Nukleinsäuresonden bevorzugt einzeln bzw. als Einzelstrang in die Zellen eindringen können, um dort an die Zielsequenz(en), sofern vorhanden, zu hybridisieren.

Neben der Behandlung mit dem Fixiermittel kann der Schritt des Fixierens der Zellen zusätzlich eine enzymatische Behandlung der Zellen bzw. des Zellhüllsystems umfassen, was insbesondere bei Gram-positiven Bakterien, bei Hefen oder bei Schimmelpilzen von Interesse sein kann. Um die Durchgängigkeit der Zellwand für das später zuzugebende spezifische Nachweisreagenz zu erhöhen, kann im Falle von Gram-positiven Bakterien beispielsweise eine Modifizierung der Peptidoglykanhülle mittels Lysozym erfolgen, oder kann im Falle von Hefen und Schimmelpilzen beispielsweise eine Modifizierung der Proteinzellwand mittels Proteasen vorgenommen werden.

Weitere Behandlungen können beispielsweise die kurzzeitige Behandlung der Zellen mittels Lösungsmitteln oder Salzsäure zur Entfernung von Wachsschichten oder dergleichen umfassen. In einer bevorzugten Ausführungsform des erfindungsgemäßen Verfahrens führt der Schritt des Fixierens der Zellen dazu, dass die fixierten Zellen nicht mehr lebensfähig sind und dennoch intakt, bevorzugt morphologisch intakt vorliegen.

Nachdem die Zellen mit einem geeigneten Fixiermittel fixiert und gegebenenfalls zusätzlich mit einem Enzym oder dergleichen behandelt worden sind, werden die fixierten Zellen aus der Probe abgetrennt. Hierzu wird die Probe zweckmäßigerweise zentrifugiert, woraufhin die sedimentierten Zellen der weiteren Aufarbeitung zugeführt werden und der Überstand verworfen wird. Andere Methoden zur Abtrennung der fixierten Zellen aus der Probe sind einem Fachmann auf dem Gebiet der mikrobiellen Diagnostik wohlbekannt und können entsprechend den jeweiligen Gegebenheiten in geeigneter Weise zur Anwendung gelangen.

Um eine Quantifizierung einzelner Zellen in einem Durchflusszytometer zu ermöglichen, ist es im Rahmen des erfindungsgemäßen Verfahrens erforderlich, dass die fixierten Zellen vor dem Inkontaktbringen der Zellen mit dem für den jeweils nachzuweisenden Mikroorganismus spezifischen Nachweisreagenz mit einem chemischen Homogenisierungsmittel versetzt und anschließend getrocknet werden. Der Begriff "chemisches Homogenisierungsmittel", wie er hierin verwendet wird, bezeichnet dabei ein chemisches Reagenz, welches die Aggregation von fixierten Zellen während der nachfolgenden Trocknung verhindert, beispielsweise indem die Ausbildung kovalenter Bindungen, ionischer Wechselwirkungen, oder dergleichen zwischen den Zellwänden zweier oder mehrerer fixierter Zellen unterbunden wird. Durch die Vereinzelung der Zellen kann sodann eine Quantifizierung einzelner Zellen in einem Durchflusszytometer erfolgen.

Erfindungsgemäß beinhaltet das chemische Homogenisierungsmittel (a) ein Monosaccharid oder ein Disaccharid, (b) ein Polyol, und (c) Wasser. Das Monosaccharid bzw. Disaccharid kann dabei natürlichen oder synthetischen Ursprungs sein, wobei als Monosaccharid insbesondere eine in D-Form oder L-Form vorliegende Tetrose, Pentose oder Hexose, wie beispielsweise Erythrose, Threose, Ribose, Arabinose, Lyxose, Xylose, Allose, Altrose, Galactose, Glucose, Gulose, Idose, Mannose, Talose und Fructose, Anwendung finden kann. Als Disaccharid kann insbesondere eine Verbindung ausgewählt aus der Gruppe bestehend aus Gentiobiose, Isomaltose, Lactose, Lactulose, Maltose, Maltulose, Raffinose, Saccharose und Trehalose eingesetzt werden, obgleich die Erfindung nicht auf die Verwendung dieser Substanzen beschränkt ist. Stärker bevorzugt wird im Rahmen der Erfindung ein Monosaccharid oder Disaccharid ausgewählt aus der Gruppe bestehend aus Fructose, Galactose, Glucose und Saccharose verwendet, und am stärksten bevorzugt Glucose.

Als weiterer Bestandteil des erfindungsgemäß eingesetzten chemischen Homogenisierungsmittels dient ein Polyol, wobei der Begriff "Polyol", wie er im Rahmen der vorliegenden Anmeldung verwendet wird, eine niedermolekulare organische Substanz mit mindestens zwei alkoholischen Hydroxygruppen bezeichnet und vorstehend definierte Monosaccharide und Disaccharide ausschließt. Das Polyol kann natürlichen oder synthetischen Ursprungs sein, und kann im Falle der Anwesenheit eines Stereozentrums oder mehrerer Stereozentren sowohl in der D-Form als auch in der L-Form vorliegen. Beispiele für erfindungsgemäß einzusetzende Polyole umfassen Ethylenglykol, Glycerin, Inositol, Isomalt, Mannitol, Pentaerythritol, Sorbitol und Xylitol, sind jedoch nicht auf diese beschränkt. Stärker bevorzugt wird im Rahmen der Erfindung ein Polyol ausgewählt aus der Gruppe bestehend aus Ethylenglykol, Glycerin, Mannitol und Sorbitol verwendet, wobei Glycerin am stärksten bevorzugt ist.

Als dritten Bestandteil umfasst das chemische Homogenisierungsmittel Wasser. Hierbei wird die Menge an Wasser üblicherweise derart eingestellt, dass die Menge an Monosaccharid oder Disaccharid in dem chemischen Homogenisierungsmittel etwa im Bereich von 10 bis 70 Gew.-%, bevorzugt etwa 20 bis 60 Gew.-%, stärker bevorzugt etwa 30 bis 50 Gew.-%, und am stärksten bevorzugt etwa 35 bis 45 Gew.-% liegt, bezogen auf das Gesamtgewicht des chemischen Homogenisierungsmittels. Die Menge an Polyol in dem chemischen Homogenisierungsmittel liegt üblicherweise im Bereich von etwa 5 bis 50 Gew.-%, bevorzugt etwa 10 bis 40 Gew.-%, stärker bevorzugt etwa 10 bis 30 Gew.-%, und am stärksten bevorzugt etwa 15 bis 25 Gew.-%, bezogen auf das Gesamtgewicht des chemischen Homogenisierungsmittels.

Nachdem die fixierten Zellen unter Einsatz des Homogenisierungsmittels homogenisiert worden sind, werden die Zellen in geeigneter Weise getrocknet, beispielsweise in einem Ofen oder Trockenschrank bei einer Temperatur im Bereich von 40 bis 90°C, bevorzugt bei einer Temperatur im Bereich von 60 bis 80°C. Anschließend werden die auf diese Weise erhaltenen getrockneten Zellen mit einem für den jeweils nachzuweisenden Mikroorganismus spezifischen Nachweisreagenz in Kontakt gebracht. Im Einzelnen werden die getrockneten Zellen hierzu typischerweise in ein geeignetes Reaktionsgefäß eingebracht und sodann zunächst mit einer Lösung mindestens einer für den Mikroorganismus spezifischen, fluoreszenzmarkierten Nukleinsäuresonde, d.h. einer mit einem Fluoreszenzfarbstoff markierten Nukleinsäuresonde, versetzt, um ein erstes Reaktionsgemisch bereitzustellen. Dieses erste Reaktionsgemisch wird sodann unter geeigneten Bedingungen inkubiert, um eine Bindung der mindestens einen fluoreszenzmarkierten Nukleinsäuresonde an die korrespondierende Zielnukleinsäuresequenz in den Zellen des nachzuweisenden Mikroorganismus zu bewirken.

Im Anschluss an diesen Schritt wird das erste Reaktionsgemisch mit einer Lösung mindestens einer quenchermarkierten Nukleinsäuresonde, d.h. einer mit einem die Fluoreszenz des Fluoreszenzfarbstoffs der fluoreszenzmarkierten Nukleinsäuresonde zumindest teilweise löschendem Quencher markierten Nukleinsäuresonde, in Kontakt gebracht, um ein zweites Reaktionsgemisch bereitzustellen, wobei die quenchermarkierte Nukleinsäuresonde eine Nukleinsäuresequenz aufweist, welche im Wesentlichen komplementär, und bevorzugt revers komplementär zu der Nukleinsäuresequenz der fluoreszenzmarkierten Nukleinsäuresonde ist. Das zweite Reaktionsgemisch wird sodann wiederum unter geeigneten Bedingungen inkubiert, um eine Bindung der nicht an die Zielnukleinsäuresequenz gebundenen Moleküle der fluoreszenzmarkierten Nukleinsäuresonde an die quenchermarkierte Nukleinsäuresonde zu bewirken und somit die Fluoreszenz von eventuell freier fluoreszenzmarkierter Nukleinsäuresonde zumindest teilweise zu löschen.

Dies bedeutet einerseits, dass im Rahmen des Verfahrens der vorliegenden Erfindung die Lösung der mindestens einen quenchermarkierten Nukleinsäuresonde direkt der Lösung der mindestens einen fluoreszenzmarkierten Nukleinsäuresonde zugegeben wird, womit eine vorherige Abtrennung eventuell überschüssiger fluoreszenzmarkierter Nukleinsäuresonde mittels eines Waschschrittes vermieden werden kann. Ferner tragen nur diejenigen fluoreszenzmarkierten Nukleinsäuresonden, die an die Zielnukleinsäuresequenz (d.h. in den Zellen des spezifisch nachzuweisenden Mikroorganismus) gebunden haben, zum Fluoreszenzsignal bei. Das Signal der freien fluoreszenzmarkierten Nukleinsäuresonden wird hingegen durch Hybridisierung mit der quenchermarkierten Nukleinsäuresonde weitestgehend ausgelöscht. Durch diesen Reaktionsmechanismus wird ein einstufiges Testsystem möglich.

Was das Mengenverhältnis der beiden Nukleinsäuresonden, d.h. der fluoreszenzmarkierten Nukleinsäuresonde und der quenchermarkierten Nukleinsäuresonde, betrifft, so hängt dieses von den Bedingungen der konkreten Ausführungsform des erfindungsgemäßen Verfahrens ab und kann mittels Routineuntersuchungen leicht von einem Fachmann bestimmt werden. Allerdings impliziert die geforderte Auslöschung der Fluoreszenz der überschüssigen, d.h. nicht an die Zielnukleinsäuresequenz gebundenen fluoreszenzmarkierten Nukleinsäuresonde durch die quenchermarkierte Nukleinsäuresonde, dass das Mengenverhältnis von quenchermarkierter Nukleinsäuresonde zu fluoreszenzmarkierter Nukleinsäuresonde mindestens 1:1 beträgt, und bevorzugt einen Überschuss an quenchermarkierter Nukleinsäuresonde vorsieht.

Was die Inkubationsbedingungen für das erste und zweite Reaktionsgemisch betrifft, so können die Inkubationszeit und die Inkubationstemperatur von einem Fachmann auf dem Gebiet der mikrobiellen Analytik anhand der Länge und dem GC Gehalt der Nukleinsäuresequenzen bestimmt und in einfachen Optimierungsverfahren überprüft werden. Allerdings beträgt die Inkubationstemperatur für das nach Zugabe der Lösung der fluoreszenzmarkierten Nukleinsäuresonde zu den getrockneten Zellen erhaltene erste Reaktionsgemisch bevorzugt etwa 60 bis 120 min, und besonders bevorzugt etwa 80 bis 100 min, während die Inkubationstemperatur bevorzugt etwa 30°C bis 50°C, und besonders bevorzugt etwa 40°C beträgt. Die Inkubationstemperatur für das nach Zugabe der Lösung der quenchermarkierten Nukleinsäuresonde zu dem ersten Reaktionsgemisch erhaltene zweite Reaktionsgemisch beträgt bevorzugt etwa 5 bis 30 min, und besonders bevorzugt etwa 10 bis 20 min, während die Inkubationstemperatur wiederum bevorzugt etwa 30°C bis 50°C, und besonders bevorzugt etwa 40°C beträgt.

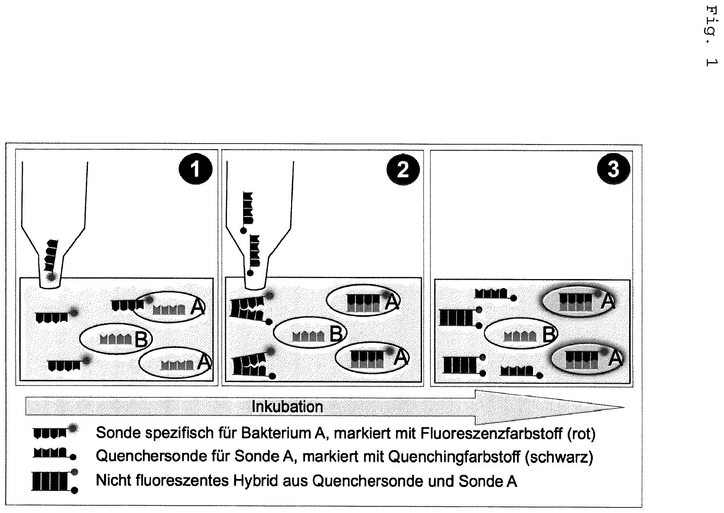

Fig. 1 veranschaulicht schematisch das dem erfindungsgemäßen Verfahren zugrundeliegende Reaktionsgeschehen, wobei in dem konkreten Beispiel sowohl die fluoreszenzmarkierte Nukleinsäuresonde als auch die quenchermarkierte Nukleinsäuresonde als einzelsträngiges DNA-Molekül vorliegt. Im Einzelnen diffundiert die fluoreszenzmarkierte Nukleinsäuresonde nach ihrer Zugabe zu den in geeigneter Weise fixierten und getrockneten Zellen zu ihrer Zielsequenz und bindet daran. Als Zielmolekül in den Zellen des nachzuweisenden Mikroorganismus fungiert hier eine rRNA, wobei die von der fluoreszenzmarkierten Nukleinsäuresonde targetierte Sequenz der rRNA spezifisch für den nachzuweisenden Mikroorganismus ist.

Ungebundene (d.h. überschüssige) fluoreszenzmarkierte Nukleinsäuresonde wird, im Gegensatz zur klassischen FISH-Technik, bei der diese im Rahmen eines stringenten Waschschrittes entfernt werden muss, durch Zugabe der quenchermarkierten Nukleinsäuresonde abgefangen, wobei sich ein nicht mehr fluoreszierendes Nukleinsäurehybrid aus fluoreszenzmarkierter Nukleinsäuresonde und quenchermarkierter Nukleinsäuresonde ausbildet. Sofern die fluoreszenzmarkierte Nukleinsäuresonde zuvor an eine geeignete Zielnukleinsäuresequenz gebunden hat, so geht diese fluoreszenzmarkierte Nukleinsäuresonde kein Hybrid mit der quenchermarkierten Nukleinsäuresonde mehr ein.

Folglich beobachtet man nach Anregung des Fluoreszenzfarbstoffes der fluoreszenzmarkierten Nukleinsäuresonde in Fällen, in welchen die fluoreszenzmarkierte Nukleinsäuresonde an die Zielnukleinsäuresequenz in dem nachzuweisenden Mikroorganismus gebunden hat, eine Fluoreszenz, während in Fällen, in welchen die fluoreszenzmarkierte Nukleinsäuresonde nicht an die Zielnukleinsäuresequenz gebunden hat, infolge Hybridisierung der fluoreszenzmarkierten Nukleinsäuresonde mit der quenchermarkierten Nukleinsäuresonde keine Fluoreszenz auftritt. Somit ist die Fluoreszenz eines in einem Durchflusszytometer detektierten Partikels ein direktes qualitatives und quantifizierbares Signal für den Mikroorganismus, für den die fluoreszenzmarkierte Nukleinsäuresonde spezifisch ist.

Vorstehend wurde das erfindungsgemäße Verfahren anhand von Nukleinsäuresonden beschrieben, die aus Desoxyribonukleotiden bestehen und daher auch als DNS-Sonden bezeichnet werden können. Es versteht sich jedoch, dass grundsätzlich auch andere Nukleinsäuresonden verwendet werden können, sofern diese Nukleinsäuresonden das vorstehend beschriebene Verhalten bezüglich der Ausbildung von Hybriden in dem nachzuweisenden Mikroorganismus zeigen.

Im Rahmen der vorliegenden Erfindung ist die fluoreszenzmarkierte Nukleinsäuresonde und/oder die quenchermarkierte Nukleinsäuresonde vorzugsweise eine einzelsträngige Nukleinsäuresonde. Allerdings ist es im Rahmen der vorliegenden Erfindung auch möglich, dass die besagten Nukleinsäuresonden einzeln und unabhängig voneinander doppelsträngig ausgebildet sind. In Fällen, in welchen zumindest eine der Nukleinsäuresonden doppelsträngig ausgebildet ist, ist es indessen bevorzugt, dass lediglich ein Teil der Sequenz der fluoreszenzmarkierten Nukleinsäuresonde bzw. ein Teil der Sequenz der quenchermarkierten Nukleinsäuresonde doppelsträngig ausgebildet ist. Das Ausmaß der Ausbildung eines Doppelstranges in den jeweiligen Sonden ist von den Erfordernissen der Hybridisierung mit der Zielnukleinsäuresequenz und insbesondere der Hybridisierung mit der jeweils komplementären Sonde abhängig.

Eine jede der im Rahmen der vorliegenden Erfindung zu verwendenden Nukleinsäuresonden, und insbesondere die fluoreszenzmarkierte Nukleinsäuresonde, ist bevorzugt als DNS-Sonde, RNS-Sonde, PNS-Sonde oder LNS-Sonde, oder als Kombinationen von zwei oder mehreren hiervon ausgebildet. Die Ausgestaltung bzw. Auswahl der die jeweiligen Nukleinsäuresonden ausbildenden Nukleotide ist im Rahmen des Könnens des Fachmanns auf dem Gebiet der Erfindung und kann durch Routineverfahren und -überlegungen im Lichte der hierin gegebenen Offenbarung und insbesondere des der vorliegenden Erfindung zugrundeliegenden mutmaßlichen Mechanismus erfolgen.

Wie vorstehend beschrieben, ist die fluoreszenzmarkierte Nukleinsäuresonde spezifisch für den nachzuweisenden Mikroorganismus. Die Erzeugung entsprechender Nukleinsäuresonden ist einem Fachmann auf dem Gebiet der mikrobiellen Analytik bekannt und u.a. in DE 10 2010 012 421 A1 näher beschrieben. Die Spezifität wird vorzugsweise über den Homologiegrad zwischen der Nukleinsäuresonde und deren Zielnukleinsäuresequenz bestimmt. Bevorzugt beträgt der Homologiegrad mindestens 70%, stärker bevorzugt mindestens 80%, noch stärker bevorzugt mindestens 95%, und am stärksten bevorzugt mindestens 96%, 97%, 98%, 99% oder 100%. In einer Ausführungsform ist die Nukleinsäuresonde damit innerhalb der vorgegebenen Homologiewerte im Wesentlichen identisch mit der Zielnukleinsäuresequenz.

Alternativ kann die Nukleotidsequenz der fluoreszenzmarkierten Nukleinsäuresonde im Wesentlichen revers komplementär zu der Zielnukleinsäuresequenz sein. Auch dann gelten die vorstehend genannten Homologiewerte als Ausmaß der Identität bzw. Komplementarität der Nukleotidsequenz der Nukleinsäuresonde mit bzw. zu der Zielnukleinsäuresequenz. Alternativ kann, insbesondere im Falle einer revers komplementären Sequenz, das Ausmaß der Homologie auch durch die Bedingungen, unter denen noch eine Hybridisierung der Nukleinsäuresonde und der Zielsequenz erfolgt, definiert werden. Die fluoreszenzmarkierte Nukleinsäuresonde ist vorzugsweise revers komplementär zu der Zielsequenz, insbesondere wenn sie mit der Zielnukleinsäuresequenz unter moderaten oder stringenten Bedingungen hybridisiert wird. Entsprechende Bedingungen sind beispielsweise in WO 00/68421 A1 beschrieben.

Das hierin zu der fluoreszenzmarkierten Nukleinsäuresonde Gesagte gilt im Wesentlichen auch für die quenchermarkierte Nukleinsäuresonde, wobei es für den Fachmann offenkundig ist, dass die quenchermarkierte Nukleinsäuresonde im Wesentlichen revers komplementär zu der fluoreszenzmarkierten Nukleinsäuresonde ist und das Ausmaß der Komplementarität zwischen der quenchermarkierten Nukleinsäuresonde und der fluoreszenzmarkierten Nukleinsäuresonde in gleicher Weise definiert werden kann wie das Ausmaß der Komplementarität zwischen der Zielnukleinsäuresequenz in dem nachzuweisenden Mikroorganismus und der fluoreszenzmarkierten Nukleinsäuresonde.

Zielnukleinsäuresequenzen, die den spezifischen Nachweis eines Mikroorganismus erlauben, sind im Fachbereich bekannt, wobei beispielhaft auf die Publikationen Clementino et al. (J. Appl. Microbiol. 103 (2007), 141-151), Ni et al. (FEMS Microbiol. Lett. 270 (2007), 58-66), Leaw et al. (J. Clin. Microbiol. 45 (2007), 2220-2229) und Bhardwaj et al. (J. Med. Microbiol. 56 (2007), 185-189) verwiesen wird. Bevorzugte Zielnukleinsäuresequenzen sind in diesem Zusammenhang insbesondere 16S rRNS, 23S rRNS, 18S rRNS, tRNS, EF-Tu, mRNS 16S-23S rRNA-Spacer und 23S-5S rRNA-Spacer, wobei 16S rRNS und 23S rRNS besonders bevorzugt sind.

Die Länge der fluoreszenzmarkierten Nukleinsäuresonde und der quenchermarkierten Nukleinsäuresonde beträgt unabhängig voneinander etwa 15 bis 31 Nukleotide, bevorzugt etwa 17 bis 25 Nukleotide, stärker bevorzugt etwa 17 bis 23 Nukleotide, und am stärksten bevorzugt 17 oder 18 Nukleotide. In einer bevorzugten Ausführungsform sind die fluoreszenzmarkierte Nukleinsäuresonde und die quenchermarkierte Nukleinsäuresonde im Wesentlichen gleich lang. Was die Auswahl der Länge der beiden Nukleinsäuresonden betrifft, so wird im Übrigen auf die in DE 10 2010 012 421 A1 genannten Kriterien verwiesen.

Beide Nukleinsäuresonden können weitere Nukleotide umfassen, als es zur Ausbildung der vorstehend genannten Längen erforderlich ist, wobei diese weiteren Nukleotide jedoch bevorzugt nicht zur Ausbildung einer doppelsträngigen Struktur beitragen oder an dieser beteiligt sind, wenn sich die fluoreszenzmarkierte Nukleinsäuresonde mit der quenchermarkierten Nukleinsäuresonde basenpaart, wobei die Basenpaarung vorzugsweise eine Watson-Crick-Basenpaarung ist und ein hybridisierter Komplex ausgebildet wird. In einer Ausführungsform ist die Länge der beiden Nukleinsäuresonden und der zueinander komplementäre Bereich gleich lang.

Ein Fluoreszenzfarbstoff, hierin auch Fluorophor bezeichnet, ist ein Molekül, welches Licht bei einer charakteristischen Wellenlänge, idealerweise an seinem Absorptionsmaximum, absorbiert bzw. davon energetisch angeregt wird. Dieses Licht (Photon) wird nach einer bestimmten Zeit z.B. wieder in Form von Fluoreszenz oder als Schwingungsenergie (Wärme) emittiert. Das Fluorophor kehrt dabei in den energetisch günstigeren nicht angeregten Ausgangszustand zurück. Ein Quencher (Akzeptor) ist ein Molekül, welches Energie von einem angeregten Fluorophor (Donator oder Donor) absorbiert und damit dessen Fluoreszenzemission auslöscht.

Generell wird als Fluoreszenzquenching die Abschwächung oder die Auslöschung eines Fluoreszenzsignals bezeichnet. Für eine optimale Quenchingeffizienz ist dabei eine genaue Abstimmung von Fluoreszenzfarbstoff und korrespondierendem Quencher entscheidend (Marras et al., Methods in Molecular Biology 335 (2006), 3-16). Bei der Auswahl eines geeigneten Paares aus Fluoreszenzfarbstoff und Quencher kann dabei unterschieden werden, ob der beobachteten Auslöschung ein statisches oder ein dynamisches Quenching zugrunde liegt. Die Positionierung des Fluoreszenzfarbstoffes und des Quenchers kann in Abhängigkeit davon erfolgen, ob es sich um ein statisches oder ein dynamisches Quenching handelt.

Beim sogenannten statischen Quenching bzw. Kontaktquenching bildet sich im angeregten Zustand ein nicht fluoreszierender Komplex zwischen Fluorophor und Quencher. Donator und Akzeptor befinden sich beim statischen Quenching räumlich sehr nahe (≤ 20 Ä). Die Moleküle interagieren dabei direkt durch einen Protonen-gekoppelten Elektronentransfer mittels der Ausbildung von Wasserstoffbrückenbindungen (Förster, Ann. Phys. 2 (1948), 55-75; Lakowicz, Principles of Fluorescence Spectroscopy, Kluwer Academic/Plenum Publishers, New York, 1999).

Es ist jedoch auch möglich, dass die Methode des dynamischen Quenchings, das sog. FRET-Quenching (Fluorescence Resonance Energy Transfer), zur Anwendung gelangt. Dabei übertragt das angeregte Fluorophor seine Energie auf den Quencher und kehrt sodann strahlungslos in den Grundzustand zurück. Donator und Akzeptor befinden sich in einem räumlichen Abstand von etwa 40 bis 100 Å (was ca. 3 bis 30 Nukleotiden innerhalb einer doppelsträngigen DNS entspricht). Eine Voraussetzung für FRET-Quenching ist zudem, dass das Fluoreszenzemissionsspektrum des Donators mit dem Absorptionsspektrum des Akzeptors überlappt.

Um ein solches Quenching zu erzielen, ist es erfindungsgemäß bevorzugt, dass der Fluoreszenzfarbstoff der fluoreszenzmarkierten Nukleinsäuresonde am 3'-Ende oder nahe des 3'-Endes der fluoreszenzmarkierten Nukleinsäuresonde angeordnet ist und der Quencher der quenchermarkierten Nukleinsäuresonde am 5'-Ende oder nahe des 5'-Endes der quenchermarkierten Nukleinsäuresonde angeordnet ist. Alternativ kann der Fluoreszenzfarbstoff der fluoreszenzmarkierten Nukleinsäuresonde allerdings auch am 5'-Ende oder nahe des 5'-Endes der fluoreszenzmarkierten Nukleinsäuresonde angeordnet sein, während der Quencher der quenchermarkierten Nukleinsäuresonde am 3'-Ende oder nahe des 3'-Endes der quenchermarkierten Nukleinsäuresonde angeordnet ist. Für weitere Einzelheiten wird in diesem Zusammenhang auf DE 10 2010 012 421 A1 verwiesen.

Es versteht sich, dass die Auslöschung der von dem Fluoreszenzfarbstoff der fluoreszenzmarkierten Nukleinsäuresonde emittierten Fluoreszenz durch den Quencher der quenchermarkierten Nukleinsäuresonde nicht zwingend vollständig sein muss. Vielmehr ist es für die Zwecke der vorliegenden Erfindung ausreichend, wenn ein signifikanter und vom Detektorsystem (d.h. dem Durchflusszytometer) detektierbarer Signalunterschied zwischen einer mit der fluoreszenzmarkierten Nukleinsäuresonde markierten Zelle des nachzuweisenden Mikroorganismus und dem Hybridisierungskomplex aus fluoreszenzmarkierter Nukleinsäuresonde und quenchermarkierter Nukleinsäuresonde vorliegt. Dabei beträgt das Ausmaß der Auslöschung üblicherweise etwa 10 bis 90%, und bevorzugt mindestens 50%.

Die zur Herstellung der fluoreszenzmarkierten Nukleinsäuresonden verwendeten Fluoreszenzfarbstoffe sind insbesondere solche, wie sie auch im Rahmen der klassischen FISH-Technik zum Einsatz gelangen. Der Fluoreszenzfarbstoff kann beispielsweise aus der Gruppe bestehend aus FAM, TAMRA, CY3, Alexa 350, Pacific Blue, Coumarin, Cy2, Alexa 488, TET, Alexa 532, HEX, Alexa 546, TMR, Cy3.5, Alexa 568, Texas red, Alexa 594, Alexa 633, Cy5, Cy5.5 Alexa 660, Alexa 680, ATTO 490LS, Rox und Vic ausgewählt sein. Es versteht sich, dass es grundsätzlich keinerlei Beschränkung hinsichtlich der Eignung von Fluoreszenzfarbstoffen gibt, mit Ausnahme dessen, dass es gleichzeitig einen die Fluoreszenz des Fluoreszenzfarbstoffs zumindest teilweise löschenden Quencher gibt.

Hinsichtlich der Ausgestaltung bzw. der Auswahl des Quenchers bestehen ebenfalls grundsätzlich keine Beschränkungen. Es versteht sich jedoch, dass die Auswahl des Fluoreszenzfarbstoffes und des Quenchers so erfolgen muss, dass eine signifikante Löschung des Fluoreszenzsignals des Fluoreszenzfarbstoffes nach dessen Anregung entweder direkt oder indirekt erfolgt. Eine ausreichende Löschung ist hierbei dahingehend definiert, dass eine Unterscheidung zwischen dem gelöschten und dem ungelöschten Zustand möglich ist. Im Rahmen der vorliegenden Erfindung ist es somit möglich, dass der Quencher selbst ein Fluoreszenzfarbstoff ist.

Einige exemplarische Kombinationen von Fluoreszenzfarbstoff und Quencher sind in der folgenden Tabelle angegeben.

| **Fluoreszenzfarbstoff** | **Korrespondierender Quencher** |
|---|---|
| FAM | Dabcyl, BHQ-1, TAMRA |
| TAMRA | BHQ-2 |
| CY3 | BHQ-2 |

Weitere geeignete Kombinationen aus Fluoreszenzfarbstoff und Quencher sind u.a. in Marras et al. (Nucl. Acids Res. 30 (2002), e122) beschrieben, auf dessen Offenbarung hiermit explizit Bezug genommen wird.

Zur Erhöhung der Spezifität des erfindungsgemäßen Verfahrens können neben der fluoreszenzmarkierten Nukleinsäuresonde und der quenchermarkierten Nukleinsäuresonde noch sogenannte Kompetitorsonden der Reaktion zugegeben werden. Unter dem Begriff "Kompetitorsonde", wie er im Rahmen der vorliegenden Anmeldung verwendet wird, werden hierbei insbesondere Oligonukleotide verstanden, welche eventuell auftretende ungewollte Bindungen, d.h. insbesondere Bindungsstellen, der Nukleinsäuresonden, insbesondere der fluoreszenzmarkierten Nukleinsäuresonde, abdecken und dabei eine höhere Sequenzähnlichkeit zu einem nicht nachzuweisenden Mikroorganismus aufweisen als zu dem/den nachzuweisenden Mikroorganismus/Mikroorganismen.

Durch den Einsatz von Kompetitorsonden kann verhindert werden, dass die fluoreszenzmarkierte Nukleinsäuresonde an die Nukleinsäuresequenz eines nicht nachzuweisenden Mikroorganismus bindet und somit zu falsch-positiven Signalen führt. Die Kompetitorsonde ist typischerweise unmarkiert und wird vorzugsweise vor Zugabe der fluoreszenzmarkierten Nukleinsäuresonde und der quenchermarkierten Nukleinsäuresonde eingesetzt. Die Kompetitorsonde sollte im Wesentlichen komplementär sein zu einer Zielnukleinsäuresequenz von einem oder mehreren nicht nachzuweisenden Mikroorganismen.

Bei der Kompetitorsonde, wie sie im Rahmen der vorliegenden Erfindung verwendet werden kann, kann es sich um eine DNA-oder RNA-Sequenz handeln, die in der Regel zwischen 12 und 100 Nukleotide umfasst, bevorzugt zwischen 15 und 50 Nukleotide, und besonders bevorzugt zwischen 17 und 25 Nukleotide. Durch die Auswahl einer definierten Sequenz kann die Hybridisierung der fluoreszenzmarkierten Nukleinsäuresonde bzw. der quenchermarkierten Nukleinsäuresonde an die Nukleinsäuresequenz einer taxonomischen Einheit oder künstlich zusammengestellten Gruppe von Mikroorganismen abgeblockt werden.

Komplementarität zu der abzublockenden Nukleinsäuresequenz sollte bei einer Kompetitorsonde von 15 Nukleotiden über 100% der Sequenz gegeben sein. Bei Kompetitorsonden mit mehr als 15 Nukleotiden sind je nach Länge ein bis mehrere Fehlpaarungsstellen erlaubt. Derartige Kompetitorsonden sind beispielsweise in der internationalen Patentanmeldung WO 2005/031004 A2 beschrieben, auf deren Offenbarung hiermit Bezug genommen wird.

Es ist im Rahmen der vorliegenden Erfindung, dass neben dem Paar aus einer fluoreszenzmarkierten Nukleinsäuresonde und einer quenchermarkierten Nukleinsäuresonde (erstes Nukleinsäuresondenpaar) noch mindestens ein weiteres Paar aus einer fluoreszenzmarkierten Nukleinsäuresonde und einer hierauf jeweils abgestimmten quenchermarkierten Nukleinsäuresonde (zweites Nukleinsäuresondenpaar, drittes Nukleinsäuresondenpaar, etc.) verwendet werden kann. Die Ausgestaltung eines jeden weiteren Nukleinsäuresondenpaars erfolgt entsprechend dem ersten Nukleinsäuresondenpaar, wobei die fluoreszenzmarkierte Nukleinsäuresonde des zweiten, dritten, etc. Nukleinsäuresondenpaars jeweils eine andere Zielnukleinsäuresequenz in dem nachzuweisenden Mikroorganismus adressiert als die fluoreszenzmarkierte Nukleinsäuresonde des ersten Nukleinsäuresondenpaars.

Dies bedeutet, dass in einer bevorzugten Variante des erfindungsgemäßen Verfahrens zunächst in Schritt (d) mehrere, für den nachzuweisenden Mikroorganismus jeweils spezifische fluoreszenzmarkierte Nukleinsäuresonden mit unterschiedlichen Nukleinsäuresequenzen in Form einer einzelnen Lösung oder mehrerer Lösungen zu den fixierten und getrockneten Zellen zugegeben werden, um parallel mehrere fluoreszenzmarkierte Nukleinsäuresonden an Zielnukleinsäuresequenzen des nachzuweisenden Mikroorganismus zu hybridisieren, und sodann in Schritt (f) eine der Anzahl der fluoreszenzmarkierten Nukleinsäuresonden entsprechende Anzahl an unterschiedlichen quenchermarkierten Nukleinsäuresonden in Form einer einzelnen Lösung oder mehrerer Lösungen zugegeben wird, um die unterschiedlichen fluoreszenzmarkierten Nukleinsäuresonden abzufangen und damit deren Fluoreszenzen zumindest teilweise zu löschen.

In einer weiteren Ausgestaltung des erfindungsgemäßen Verfahrens enthält die zu analysierende Probe mehr als einen Mikroorganismus, d.h. mindestens zwei unterschiedliche Mikroorganismen, welche parallel nachgewiesen werden sollen. In diesem Fall gilt bevorzugt, dass die fluoreszenzmarkierte Nukleinsäuresonde des zweiten Nukleinsäuresondenpaars spezifisch für den zweiten nachzuweisenden Mikroorganismus ist, die fluoreszenzmarkierte Nukleinsäuresonde des dritten Nukleinsäuresondenpaars spezifisch für den dritten nachzuweisenden Mikroorganismus ist, etc. Allerdings besteht selbstverständlich auch hier die Möglichkeit, dass zum Nachweis eines jeden nachzuweisenden Mikroorganismus jeweils mehr als nur eine hierfür spezifische fluoreszenzmarkierte Nukleinsäuresonde eingesetzt wird.

Nachdem die Fluoreszenz eventuell überschüssiger fluoreszenzmarkierter Nukleinsäuresonde(n) zumindest teilweise gelöscht worden ist, wird das zweite Reaktionsgemisch zur Detektion der Fluoreszenz, welche von den die fluoreszenzmarkierte Nukleinsäuresonde(n) beinhaltenden Zellen des nachzuweisenden Mikroorganismus emittiert wird, in ein handelsübliches Durchflusszytometer eingebracht und in diesem analysiert. Da das zweite Reaktionsgemisch als solches (d.h. ohne weitere Aufarbeitung und Waschschritte) in das Durchflusszytometer eingebracht werden kann, verringert sich der Arbeitsaufwand und können eventuelle Probenverluste verhindert werden. Insofern betrifft die Erfindung in einem zweiten Aspekt die Verwendung eines Durchflusszytometers in dem Verfahren des ersten Aspekts, wie in Anspruch 14 definiert ist.

Die von den Zellen emittierte Fluoreszenz kann direkt zur Quantifizierung der Anzahl der Zellen, insbesondere der Ganzzellen, des nachzuweisenden Mikroorganismus verwendet werden. Die bei dieser Messung erhaltenen Messwerte werden sodann in Form von Histogrammen oder Dotplots auf dem Computer visualisiert und erlauben die zuverlässige Aussage über die Art und die Menge der in der Probe enthaltenen Mikroorganismen. Somit erlaubt das erfindungsgemäße Verfahren einen direkten Nachweis sowie eine Quantifizierung eines Mikroorganismus oder mehrerer Mikroorganismen als ganze Zellen im Rahmen einer Ganzzellhybridisierung.

Wesentliche Vorteile des erfindungsgemäßen Verfahrens sind somit die sehr leichte Handhabung sowie weiterhin Schnelligkeit, Reproduzierbarkeit, Zuverlässigkeit und Objektivität, mit der der spezifische Nachweis von Mikroorganismen in einer Probe möglich ist. Ein weiterer Vorteil besteht darin, dass nun erstmals das vorteilhafte Verfahren der in-situ-Hybridisierung in Lösung so durchgeführt werden kann, dass auf jegliche Waschschritte verzichtet werden kann. Dies vereinfacht die Handhabung des Verfahrens und verkürzt somit die aufzuwendende Arbeitszeit für die Präparationsschritte. Da zusätzlich der gesamte Ablauf des Verfahrens in nur einem Reaktionsgefäß stattfinden kann, sind Verluste beim Überführen in neue Reaktionsgefäße ausgeschlossen, so dass eine präzisere Quantifizierung möglich ist.

Die vorliegende Erfindung wird nun anhand der folgenden Beispiele, Vergleichsbeispiele und Figuren weiter erläutert.

### Beispiel 1

Verfahren zum spezifischen Nachweis von Mikroorganismen am Beispiel des Nachweises von Alicyclobaccillus spec. in Fruchtsaftgetränken

Eine zu untersuchende Probe eines Orangensaftkonzentrats wurde in geeigneter Weise für mindestens 48 h kultiviert. Anschließend wurden 0.9 ml der Kultur in ein geeignetes Reaktionsgefäß überführt und mit 0.9 ml eines Fixierungsmittels versetzt, welches 80%-iges Ethanol enthielt.

Die fixierten Zellen wurden durch Zentrifugation sedimentiert (4000 x g, 5 min, Raumtemperatur), sodann mit 20 µl eines Homogenisierungsmittels versetzt, welches durch Mischen einer 50 Gew.-% wässrigen Glucoselösung mit Glycerin im Gewichtsverhältnis 8:2 erhalten worden war, und abschließend für 20 Minuten bei 80°C im Ofen eingetrocknet.

Anschließend wurden die eingetrockneten und homogenisierten Zellen in einem Reaktionsgefäß mit 35 µl einer Hybridisierungslösung versetzt, in welcher 20 ng einer für Alicyclobacillus spec. spezifischen, mit FAM als Fluoreszenzfarbstoff markierten Nukleinsäuresonde in einem wässrigen Puffer (Lösung von 0.9 M NaCl und 0.02 M Tris-HCl (pH 8.0) in einem Gemisch aus 65 Gew.-% Wasser und 35 Gew.-% Formamid) gelöst waren. Die fluoreszenzmarkierte Nukleinsäuresonde besaß eine Länge von 20 Nukleotiden und wies einen Homologiegrad zur Zielnukleinsäuresequenz von 100% auf.

Nachdem das auf diese Weise erhaltene Reaktionsgemisch für 1.5 h bei 40°C inkubiert worden war, wurden dem Reaktionsgemisch 35 µl einer Quencherlösung hinzugefügt, in welcher 20 ng einer mit BHQ1 als Quencher markierten Nukleinsäuresonde in einem wässrigen Puffer (Lösung von 0.14 M NaCl und 0.04 M Tris-HCl (pH 8.0) in Wasser) gelöst waren. Die quenchermarkierte Nukleinsäuresonde besaß dabei eine Länge von 20 Nukleotiden und wies einen Homologiegrad zur Nukleinsäuresequenz der fluoreszenzmarkierten Nukleinsäuresonde von 100% auf.

Das auf diese Weise erhaltene Reaktionsgemisch wurde für weitere 15 Minuten bei 40°C inkubiert und sodann ohne weitere Aufarbeitung in ein Durchflusszytometer (Modell CyFlow® Cube6 der Firma Sysmex Deutschland GmbH) eingebracht und in diesem bei einer Durchflussgeschwindigkeit von 0.5 µl/sec analysiert und ausgewertet.

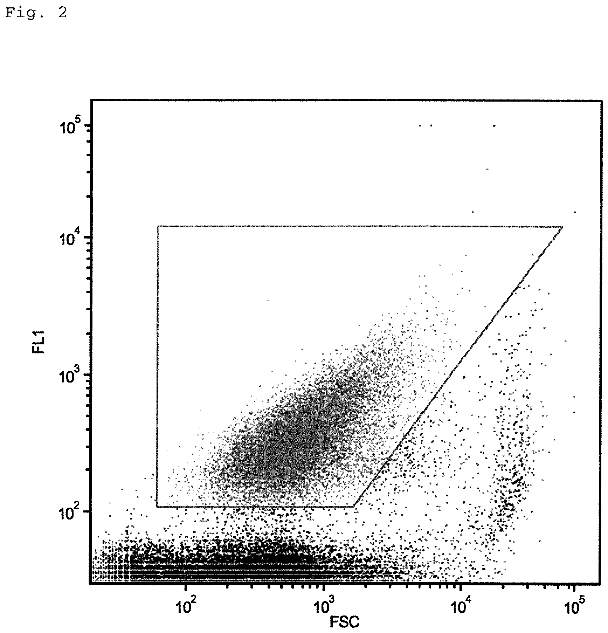

Fig. 2 zeigt das Ergebnis der Messung. Das Fluoreszenzsignal der Zellen ist in dem Dotplot-Diagramm in Y-Richtung gegen die Streuung, welche ein Maß für die Größe des Partikels ist, in X-Richtung aufgetragen.

### Vergleichsbeispiel 1

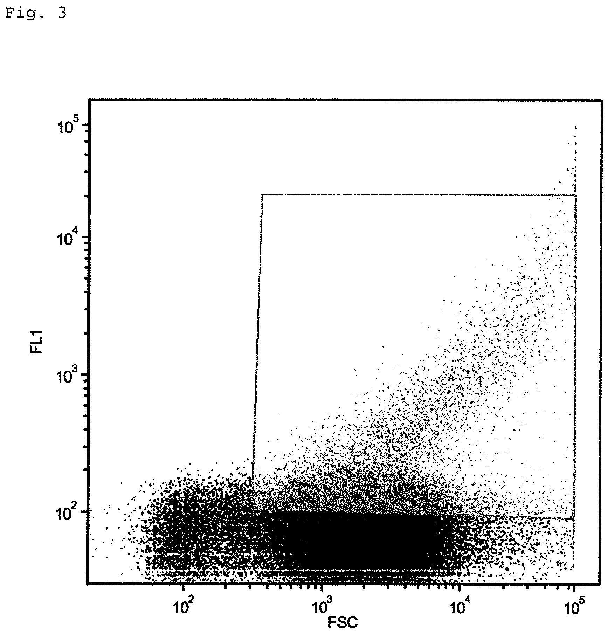

Das vorstehend beschriebene Beispiel 1 wurde wiederholt, mit Ausnahme dessen, dass vor der Trocknung der fixierten Zellen kein Homogenisierungsmittel zugegeben wurde. Die Ergebnisse sind in Fig. 3 dargestellt.

Aufgrund des Fehlens des Homogenisierungsmittels wurden nur wenige, aber dafür sehr große Partikel, die jeweils aus Clustern einer unterschiedlichen Anzahl von zusammenhängenden einzelnen Zellen bestanden, detektiert. Durch die Clusterbildung war eine Quantifizierung der Zellzahl anhand der Anzahl der im Durchflusszytometer detektierten Partikel nicht mehr möglich.

Ein weiterer Nachteil der Clusterbildung ergibt sich aus der stark divergierenden Verteilung der detektierten Partikel in Bezug auf die Größe (X-Achse des Dotplots) und die Intensität des detektierten Fluoreszenzsignals (Y-Achse des Dotplots), da ein Cluster, der aus einer größeren Anzahl von einzelnen Zellen gebildet wird, vom Detektor als Partikel mit einer höheren Intensität des Fluoreszenzsignals wahrgenommen wird als ein Cluster, der aus einer geringeren Anzahl von einzelnen Zellen besteht. Dementsprechend divergiert die Verteilung der detektierten Partikel im Dotplot sehr stark, was eine differenzierte Bestimmung der Organismen stark einschränkt.

### Vergleichsbeispiel 2

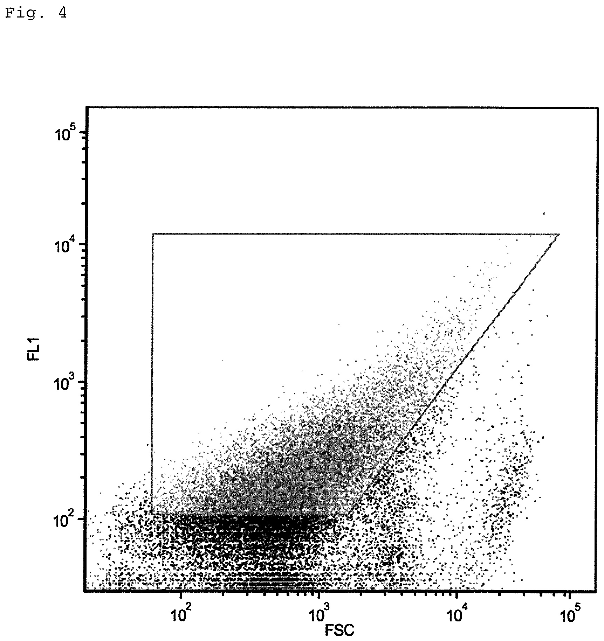

Das vorstehend beschriebene Beispiel 1 wurde Im Wesentlichen wiederholt, mit Ausnahme dessen, dass das in WO 03/083131 A1 beschriebene Verfahren angewendet und keine quenchermarkierte Nukleinsäuresonde eingesetzt wurde. Das Ergebnis ist in Fig. 4 dargestellt.

Aufgrund des Fehlens des Quenchers wurde die Fluoreszenz der ungebundenen fluoreszenzmarkierten Nukleinsäuresonden nicht gelöscht. Reste der ungebundenen fluoreszenzmarkierten Nukleinsäuresonden, die durch Waschen nicht vollständig entfernt werden konnten, trugen zu einer Erhöhung des Hintergrundrauschens bei der Messung bei und führten zu einer schlechteren Trennung des Fluoreszenzsignals vom Hintergrundrauschen.

### Beispiel 2 (nicht erfindungsgemäß)

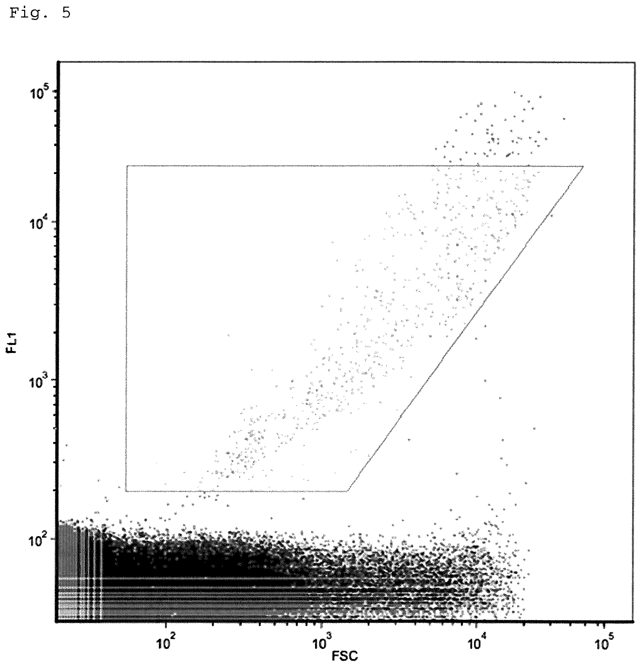

Das vorstehend beschriebene Beispiel 1 wurde wiederholt, mit Ausnahme dessen, dass anstelle des im Beispiel 1 verwendeten Homogenisierungsmittels 70%-iges Ethanol als Homogenisierungsmittel eingesetzt wurde. Die Ergebnisse sind in Fig. 5 dargestellt.

Aufgrund der Verwendung von 70%-igem Ethanol als Homogenisierungsmittel wurde eine deutliche Verbesserung der Verteilung der detektierten Partikel gegenüber dem Vergleichsbeispiel 1 erzielt, in welchem vor der Trocknung der fixierten Zellen kein Homogenisierungsmittel zugesetzt wurde.

Wie anhand einer relativ hohen Anzahl von großen Partikeln mit intensivem Fluoreszenzsignal erkennbar ist, wurden allerdings auch bei Einsatz von 70%-igem Ethanol als Homogenisierungsmittel Cluster gebildet, welche aus mehreren zusammenhängenden einzelnen Zellen bestanden.

## Patentansprüche

1. Verfahren zum Nachweis eines Mikroorganismus in einer Probe, umfassend die Schritte:
(a) Bereitstellen der Probe;
(b) Fixieren der in der Probe enthaltenen Zellen mit einem Fixiermittel und Abtrennen der hierbei erhaltenen fixierten Zellen aus der Probe, um fixierte Zellen bereitzustellen;
(c) Inkontaktbringen der fixierten Zellen mit einem chemischen Homogenisierungsmittel und Trocknen der hierbei erhaltenen homogenisierten Zellen, um getrocknete Zellen bereitzustellen, wobei das chemische Homogenisierungsmittel (i) ein Monosaccharid oder Disaccharid, (ii) ein Polyol, und (iii) Wasser umfasst;
(d) Inkontaktbringen der getrockneten Zellen mit einer Lösung einer für den nachzuweisenden Mikroorganismus spezifischen, fluoreszenzmarkierten Nukleinsäuresonde, um ein erstes Reaktionsgemisch bereitzustellen;
(e) Inkubieren des ersten Reaktionsgemischs, um eine Bindung der fluoreszenzmarkierten Nukleinsäuresonde an die korrespondierende Zielnukleinsäuresequenz in den Zellen des nachzuweisenden Mikroorganismus zu bewirken;
(f) Inkontaktbringen des ersten Reaktionsgemischs im Anschluss an Schritt (e) mit einer Lösung einer quenchermarkierten Nukleinsäuresonde, um ein zweites Reaktionsgemisch bereitzustellen, wobei die quenchermarkierte Nukleinsäuresonde einen die Fluoreszenz der fluoreszenzmarkierten Nukleinsäuresonde zumindest teilweise löschenden Quencher umfasst und eine Nukleinsäuresequenz aufweist, welche im Wesentlichen komplementär zu der Nukleinsäuresequenz der fluoreszenzmarkierten Nukleinsäuresonde ist;
(g) Inkubieren des zweiten Reaktionsgemischs, um eine Bindung der nicht an die Zielnukleinsäuresequenz in den Zellen des nachzuweisenden Mikroorganismus gebundenen Moleküle der fluoreszenzmarkierten Nukleinsäuresonde an die quenchermarkierte Nukleinsäuresonde zu bewirken; und
(h) Einbringen des zweiten Reaktionsgemischs im Anschluss an Schritt (g) in ein Durchflusszytometer, und Detektieren der Fluoreszenz, welche von den die fluoreszenzmarkierte Nukleinsäuresonde beinhaltenden Zellen des nachzuweisenden Mikroorganismus emittiert wird.

2. Verfahren nach Anspruch 1, wobei es sich bei der Probe um eine flüssige Probe handelt.

3. Verfahren nach Anspruch 1 oder 2, wobei es sich bei dem Mikroorganismus um ein Bakterium, einen Pilz, oder einen einzelligen höheren Organismus (Protozoon) handelt.

4. Verfahren nach Anspruch 3, wobei es sich bei dem Bakterium um ein Bakterium der Gattung Acinetobacter, Alicyclobacillus, Aquabacterium, Arcobacter, Bacillus, Campylobacter, Enterobacteriaceae, Escherichia, Lactobacillus, Lactococcus, Legionella, Listeria, Microthrix, Nitrobacter, Nitrosococcus, Nitrosomonas, Nitrospira, Nitrotoga, Propionibacterium, Salmonella, Shigella oder Streptococcus handelt.

5. Verfahren nach Anspruch 3, wobei es sich bei dem Pilz um einen Pilz der Gattung Aspergillus, Candida, Debaromyces, Dekkera, Penicillium, Pichia oder Saccharomyces handelt.

6. Verfahren nach einem der Ansprüche 1 bis 5, wobei es sich bei dem Monosaccharid oder Disaccharid um eine Substanz ausgewählt aus der Gruppe bestehend aus Fructose, Galactose, Glucose und Saccharose handelt.

7. Verfahren nach einem der Ansprüche 1 bis 6, wobei es sich bei dem Polyol um eine Substanz ausgewählt aus der Gruppe bestehend aus Ethylenglycol, Glycerin, Mannitol und Sorbitol handelt.

8. Verfahren nach einem der Ansprüche 1 bis 7, wobei die Zielnukleinsäuresequenz in den Zellen des nachzuweisenden Mikroorganismus aus der Gruppe bestehend aus 16S rRNS, 23S rRNS, 18S rRNS, tRNS, EF-Tu, mRNS 16S-23S rRNA-Spacer und 23S-5S rRNA-Spacer ausgewählt ist.

9. Verfahren nach einem der Ansprüche 1 bis 8, wobei die fluoreszenzmarkierte Nukleinsäuresonde (i) im Wesentlichen identisch oder (ii) im Wesentlichen revers komplementär zu der Zielnukleinsäuresequenz in den Zellen des nachzuweisenden Mikroorganismus ist.

10. Verfahren nach einem der Ansprüche 1 bis 9, wobei die fluoreszenzmarkierte Nukleinsäuresonde aus einer fluoreszenzmarkierten DNS-Sonde, RNS-Sonde, PNS-Sonde und LNS-Sonde ausgewählt ist.

11. Verfahren nach einem der Ansprüche 1 bis 10, wobei (i) der Fluroreszenzfarbstoff der fluoreszenzmarkierten Nukleinsäuresonde am 3'-Ende oder nahe des 3'-Endes der fluoreszenzmarkierten Nukleinsäuresonde angeordnet ist und der Quencher der quenchermarkierten Nukleinsäuresonde am 5'-Ende oder nahe des 5'-Endes der quenchermarkierten Nukleinsäuresonde angeordnet ist, oder (ii) der Fluroreszenzfarbstoff der fluoreszenzmarkierten Nukleinsäuresonde am 5'-Ende oder nahe des 5'-Endes der fluoreszenzmarkierten Nukleinsäuresonde angeordnet ist und der Quencher der quenchermarkierten Nukleinsäuresonde am 3'-Ende oder nahe des 3'-Endes der quenchermarkierten Nukleinsäuresonde angeordnet ist.

12. Verfahren nach einem der Ansprüche 1 bis 11, wobei in Schritt (d) mehrere, für den nachzuweisenden Mikroorganismus jeweils spezifische fluoreszenzmarkierte Nukleinsäuresonden mit unterschiedlichen Nukleinsäuresequenzen zugegeben werden, und in Schritt (f) eine der Anzahl der fluoreszenzmarkierten Nukleinsäuresonden entsprechende Anzahl an unterschiedlichen quenchermarkierten Nukleinsäuresonden zugegeben wird.

13. Verfahren nach einem der Ansprüche 1 bis 12, wobei die Probe mehr als einen Mikroorganismus enthält und mehrere unterschiedliche Mikroorganismen gleichzeitig nachgewiesen werden.

14. Verwendung eines Durchflusszytometers in einem Verfahren nach einem der Ansprüche 1 bis 13.

## Claims

1. Method for detecting a microorganism in a sample, comprising the steps of:
(a) providing the sample;
(b) fixing the cells contained in the sample using a fixing agent and separating the resulting fixed cells from the sample in order to provide fixed cells;
(c) bringing the fixed cells into contact with a chemical homogenising agent and drying the resulting homogenised cells in order to provide dried cells, wherein the chemical homogenising agent comprises (i) a monosaccharide or disaccharide, (ii) a polyol, and (iii) water;
(d) bringing the dried cells into contact with a solution of a fluorescently tagged nucleic acid probe specific to the microorganism to be detected in order to provide a first reaction mixture;
(e) incubating the first reaction mixture in order to cause binding of the fluorescently tagged nucleic acid probe to the corresponding target nucleic acid sequence in the cells of the microorganism to be detected;
(f) following step (e), bringing the first reaction mixture into contact with a solution of a quencher-tagged nucleic acid probe in order to provide a second reaction mixture, wherein the quencher-tagged nucleic acid probe comprises a quencher which at least partially quenches the fluorescence of the fluorescently tagged nucleic acid probe and has a nucleic acid sequence which is substantially complementary to the nucleic acid sequence of the fluorescently tagged nucleic acid probe;
(g) incubating the second reaction mixture in order to bring about binding of the molecules of the fluorescently tagged nucleic acid probe which are not bound to the target nucleic acid sequence in the cells of the microorganism to be detected to the quencher-tagged nucleic acid probe; and
(h) following step (g), introducing the second reaction mixture into a flow cytometer, and detecting the fluorescence emitted by the cells of the microorganism to be detected which contain the fluorescently tagged nucleic acid probe.

2. Method according to claim 1, wherein the sample is a liquid sample.

3. Method according to either claim 1 or claim 2, wherein the microorganism is a bacterium, a fungus or a unicellular higher organism (protozoan).

4. Method according to claim 3, wherein the bacterium is a bacterium of the genus Acinetobacter, Alicyclobacillus, Aquabacterium, Arcobacter, Bacillus, Campylobacter, Enterobacteriaceae, Escherichia, Lactobacillus, Lactococcus, Legionella, Listeria, Microthrix, Nitrobacter, Nitrosococcus, Nitrosomonas, Nitrospira, Nitrotoga, Propionibacterium, Salmonella, Shigella or Streptococcus.

5. Method according to claim 3, wherein the fungus is a fungus of the genus Aspergillus, Candida, Debaromyces, Dekkera, Penicillium, Pichia or Saccharomyces.

6. Method according to any of claims 1 to 5, wherein the monosaccharide or disaccharide is a substance selected from the group consisting of fructose, galactose, glucose and sucrose.

7. Method according to any of claims 1 to 6, wherein the polyol is a substance selected from the group consisting of ethylene glycol, glycerol, mannitol and sorbitol.

8. Method according to any of claims 1 to 7, wherein the target nucleic acid sequence in the cells of the microorganism to be detected is selected from the group consisting of 16S rRNA, 23S rRNA, 18S rRNA, tRNA, EF-Tu, mRNA 16S-23S rRNA spacer and 23S-5S rRNA spacer.

9. Method according to any of claims 1 to 8, wherein the fluorescently tagged nucleic acid probe is (i) substantially identical or (ii) substantially reverse complementary to the target nucleic acid sequence in the cells of the microorganism to be detected.

10. Method according to any of claims 1 to 9, wherein the fluorescently tagged nucleic acid probe is selected from a fluorescently tagged DNA probe, RNA probe, PNA probe and LNA probe.

11. Method according to any of claims 1 to 10, wherein (i) the fluorescent dye of the fluorescently tagged nucleic acid probe is arranged at the 3' end or near the 3' end of the fluorescently tagged nucleic acid probe and the quencher of the quencher-tagged nucleic acid probe is arranged at the 5' end or near the 5' end of the quencher-tagged nucleic acid probe, or (ii) the fluorescent dye of the fluorescently tagged nucleic acid probe is arranged at the 5' end or near the 5' end of the fluorescently tagged nucleic acid probe and the quencher of the quencher-tagged nucleic acid probe is located at the 3' end or near the 3' end of the quencher-tagged nucleic acid probe.

12. Method according to any of claims 1 to 11, wherein, in step (d), multiple fluorescently tagged nucleic acid probes, each specific to the microorganism to be detected, having different nucleic acid sequences are added, and, in step (f), a number of different quencher-tagged nucleic acid probes corresponding to the number of fluorescently tagged nucleic acid probes is added.

13. Method according to any of claims 1 to 12, wherein the sample contains more than one microorganism and multiple different microorganisms are detected at the same time.

14. Use of a flow cytometer in a method according to any of claims 1 to 13.

## Revendications

1. Procédé pour la mise en évidence d'un micro-organisme dans un échantillon, comprenant les étapes :
(a) de fourniture de l'échantillon ;
(b) de fixation des cellules contenues dans l'échantillon avec un agent fixateur et de séparation des cellules fixées obtenues à cette occasion de l'échantillon, afin de fournir des cellules fixées ;
c) d'amenée en contact des cellules fixées avec un agent d'homogénéisation chimique et de séchage des cellules homogénéisées obtenues à cette occasion, afin de fournir des cellules séchées, dans lequel l'agent d'homogénéisation chimique comprend (i) un monosaccharide ou disaccharide, (ii) un polyol, et (iii) de l'eau ;
(d) d'amenée en contact des cellules séchées avec une solution d'une sonde d'acide nucléique marquée par fluorescence, spécifique du micro-organisme à mettre en évidence, afin de fournir un premier mélange réactionnel ;
(e) d'incubation du premier mélange réactionnel, afin de provoquer une liaison de la sonde d'acide nucléique marquée par fluorescence à la séquence d'acide nucléique cible correspondante dans les cellules du micro-organisme à mettre en évidence ;
(f) d'amenée en contact du premier mélange réactionnel à la suite de l'étape (e) avec une solution d'une sonde d'acide nucléique marquée par un quencher, afin de fournir un deuxième mélange réactionnel, dans lequel la sonde d'acide nucléique marquée par un quencher comprend un quencher éteignant au moins en partie la fluorescence de la sonde d'acide nucléique marquée par fluorescence et présente une séquence d'acide nucléique, laquelle est sensiblement complémentaire de la séquence d'acide nucléique de la sonde d'acide nucléique marquée par fluorescence ;
(g) d'incubation du deuxième mélange réactionnel, afin de provoquer une liaison de la molécule de la sonde d'acide nucléique marquée par fluorescence non liée à la séquence d'acide nucléique cible dans les cellules du micro-organisme à mettre en évidence à la sonde d'acide nucléique marquée par un quencher ; et
(h) d'introduction du deuxième mélange réactionnel à la suite de l'étape (g) dans un cytomètre en flux, et de détection de la fluorescence, laquelle est émise par les cellules du micro-organisme à mettre en évidence comportant la sonde d'acide nucléique marquée par fluorescence.

2. Procédé selon la revendication 1, dans lequel il s'agit pour la sonde d'une sonde liquide.

3. Procédé selon la revendication 1 ou 2, dans lequel il s'agit pour le micro-organisme d'une bactérie, d'un champignon, ou d'un organisme supérieur unicellulaire (protozoaire).

4. Procédé selon la revendication 3, dans lequel il s'agit pour la bactérie d'une bactérie du genre Acinetobacter, Alicyclobacillus, Aquabacterium, Arcobacter, Bacillus, Campylobacter, Enterobacteriaceae, Escherichia, Lactobacillus, Lactococcus, Legionella, Listeria, Microthrix, Nitrobacter, Nitrosococcus, Nitrosomonas, Nitrospira, Nitrotoga, Propionibacterium, Salmonella, Shigella ou Streptococcus.

5. Procédé selon la revendication 3, dans lequel il s'agit pour le champignon d'un champignon du genre Aspergillus, Candida, Debaromyces, Dekkera, Penicillium, Pichia ou Saccharomyces.

6. Procédé selon l'une quelconque des revendications 1 à 5, dans lequel il s'agit pour le monosaccharide ou disaccharide d'une substance sélectionnée dans le groupe constitué du fructose, galactose, glucose et saccharose.

7. Procédé selon l'une quelconque des revendications 1 à 6, dans lequel il s'agit pour le polyol d'une substance sélectionnée dans le groupe constitué d'éthylène glycol, de glycérine, de mannitol et de sorbitol.

8. Procédé selon l'une quelconque des revendications 1 à 7, dans lequel la séquence d'acide nucléique cible dans les cellules du micro-organisme à mettre en évidence est sélectionnée dans le groupe constitué de ARNr 16S, ARNr 23S, ARNr 18S, ARNt, EF-Tu, ARNm espaceur ARNr 16S-23S et espaceur ARNr 23S-5S.

9. Procédé selon l'une quelconque des revendications 1 à 8, dans lequel la sonde d'acide nucléique marquée par fluorescence est (i) sensiblement identique ou (ii) sensiblement inversement complémentaire de la séquence d'acide nucléique cible dans les cellules du micro-organisme à mettre en évidence.

10. Procédé selon l'une quelconque des revendications 1 à 9, dans lequel la sonde d'acide nucléique marquée par fluorescence est sélectionnée parmi une sonde ADN, sonde ARN, sonde PNS et sonde LNS marquée par fluorescence.

11. Procédé selon l'une quelconque des revendications 1 à 10, dans lequel (i) le fluorochrome de la sonde d'acide nucléique marquée par fluorescence est disposé à l'extrémité 3' ou près de l'extrémité 3' de la sonde d'acide nucléique marquée par fluorescence et le quencher de la sonde d'acide nucléique marquée par un quencher est disposé à l'extrémité 5' ou près de l'extrémité 5' de la sonde d'acide nucléique marquée par un quencher, ou (ii) le fluorochrome de la sonde d'acide nucléique marquée par fluorescence est disposé à l'extrémité 5' ou près de l'extrémité 5' de la sonde d'acide nucléique marquée par fluorescence et le quencher de la sonde d'acide nucléique marquée par un quencher est disposé à l'extrémité 3' ou près de l'extrémité 3' de la sonde d'acide nucléique marquée par un quencher.

12. Procédé selon l'une quelconque des revendications 1 à 11, dans lequel à l'étape (d) plusieurs sondes d'acide nucléique marquées par fluorescence respectivement spécifiques du micro-organisme à mettre en évidence sont ajoutées à des séquences d'acide nucléique différentes, et à l'étape (f) un nombre de sondes d'acide nucléique différentes marquées par un quencher correspondant au nombre de sondes d'acide nucléique marquées par fluorescence est ajouté.

13. Procédé selon l'une quelconque des revendications 1 à 12, dans lequel la sonde contient plus d'un micro-organisme et plusieurs micro-organismes différents sont mis en évidence simultanément.

14. Utilisation d'un cytomètre en flux dans un procédé selon l'une quelconque des revendications 1 à 13.
